(19) Europäisches Patentamt
European Patent Office
Office européen des brevets

(11) Veröffentlichungsnummer: **0 252 873**
**A1**

(12) # EUROPÄISCHE PATENTANMELDUNG

(21) Anmeldenummer: **87730073.1**

(22) Anmeldetag: **07.07.87**

(51) Int. Cl.$^4$: **C 07 D 457/12**
A 61 K 31/48

(30) Priorität: **09.07.86 DE 3623503**

(43) Veröffentlichungstag der Anmeldung:
**13.01.88 Patentblatt 88/02**

(84) Benannte Vertragsstaaten:
**AT BE CH DE ES FR GB GR IT LI LU NL SE**

(71) Anmelder: **SCHERING AKTIENGESELLSCHAFT Berlin und Bergkamen**
**Müllerstrasse 170/178 Postfach 65 03 11**
**D-1000 Berlin 65 (DE)**

(72) Erfinder: **Sauer, Gerhard, Dr.**
**Königsbacher Zeile 41A**
**D-1000 Berlin 28 (DE)**

**Biere, Helmut, Dr.**
**Zeltinger Strasse 15**
**D-1000 Berlin 28 (DE)**

**Wachtel, Helmut, Dr.**
**Suarezstrasse 22**
**D-1000 Berlin 19 (DE)**

(54) **1-Aryl-ergolinyl-harnstoffderivate, Verfahren zu deren Herstellung sowie die Verwendung dieser Verbindungen als Arzneimittel.**

(57) Es werden Verbindungen der allgemeinen Formel I

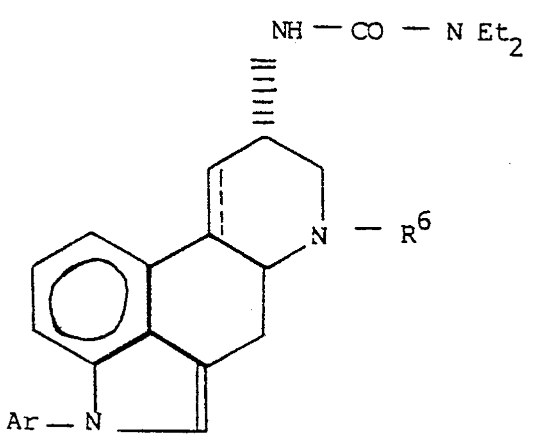

worin
Ar für einen gegebenenfalls substituierten Aryl- oder Hetarylrest steht und
$R^6$ niederes Alkyl bedeutet und
$C_9$ ═══ $C_{10}$ eine Einfach- oder Doppelbindung sein kann
sowie deren Säureadditionssalze,
beschrieben. Diese Verbindungen besitzen wertvolle pharmakologische Eigenschaften.

EP 0 252 873 A1

**Beschreibung**

1-Aryl-ergolinyl-harnstoffderivate, Verfahren zu deren Herstellung sowie die Verwendung dieser Verbindungen als Arzneimittel

Die Erfindung betrifft 1-Aryl-ergolinyl-harnstoffderivate, ihre Herstellung sowie ihre Verwendung als Arzneimittel.

Die erfindungsgemäßen Verbindungen haben die allgemeine Formel I

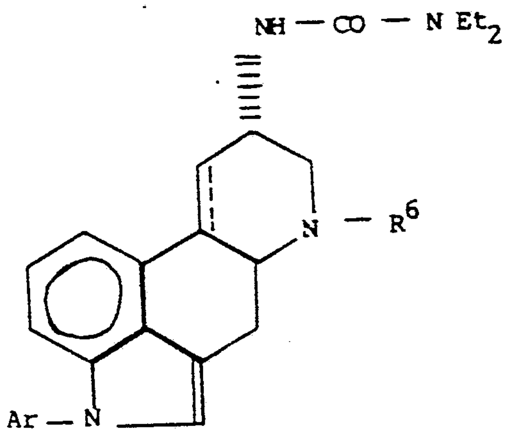

I

worin
Ar für einen gegebenenfalls substituierten Aryl- oder Hetarylrest steht und
$R^6$ niederes Alkyl bedeutet und
$C_9$ ===== $C_{10}$ eine Einfach- oder Doppelbindung sein kann sowie deren Säureadditionssalze.

Der Arylrest ist ein gegebenenfalls mono- oder mehrfach substituierter, ein- oder mehrkerniger, homo- oder heteroaromatischer Rest, wobei als Substituenten z.B. Halogen, Hydroxy, Cyano oder Trifluormethyl-, Nitro-, Amino-, Acylamino-, Alkylamino-, Alkyl-, Acyloxy-, Alkoxy-, Alkoxycarbonyl-, Hydroxycarbonyl-, Aminocarbonyl-, Formyl-, Hydroxyalkyl-, Alkoxyalkyl-, Acloxyalkyl-, Aminoalkyl-, mono- oder di-Alkylaminoalkylgruppen oder Acylaminoalkyl in Frage kommen.

Der nicht heterocyclische Arylrest kann 6-12 C-Atome enthalten wie beispielsweise Phenyl oder 1- oder 2-Naphthyl. Als Heteroaromaten kommen 5-oder 6-Ring Heteroaromaten in Betracht, die 1-3 Heteroatome enthalten können wie N-, O- und S-Atome.Falls die Aromaten oder Heteroaromaten substituiert sind, haben sie bevorzugt 1-2 Substituenten.

Bevorzugt steht Aryl für eine Phenylgruppe, die mono- oder disubstituiert sein kann in o-, m- oder p-Stellung wie beispielsweise für den Rest

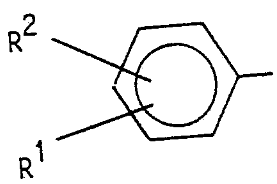

worin
$R^1$ und $R^2$ jeweils Wasserstoff, Trifluormethyl, Nitro, Cyano, Halogen, Formyl, Aminocarbonyl, niederes Alkyl,
$NR^3R^4$ , wobei $R^3$ und $R^4$ jeweils Wasserstoff, niederes Alkyl oder niederes Acyl darstellen,
$OR^5$ , wobei $R^5$ Wasserstoff, niederes Alkyl oder niederes Acyl bedeutet,
$COOR^7$ , wobei $R^7$ Wasserstoff oder niederes Alkyl ist,
$CH_2$ $OR^8$ , wobei $R^8$ Wasserstoff, niederes Alkyl oder niederes Acyl ist
oder
$CH_2$ $NR^9$ $R^{10}$ , wobei $R^9$ und $R^{10}$ jeweils Wasserstoff, niederes Alkyl oder niederes Acyl darstellt,
bedeuten.

Als Heteroaromaten sind Pyrrol-, Furan-, Imidazol-, Thiophen-, Oxazol- und bevorzugt Pyridin-, Pyrimidin-, Pyrazin-, oder Thiazolreste geeignet, die gegebenenfalls mit $R^1$ und/oder $R^2$ substituiert sein können.

Unter Halogen ist Fluor, Chlor, Brom und Jod, insbesondere Fluor und Brom, zu verstehen.

Unter niederem Alkyl sind sowohl gerad- als auch verzweigtkettige Reste mit $C_1$ - $C_6$ Kohlenstoffatomen zu verstehen. Beispielsweise seien die bevorzugten $C_{1-4}$-Alkylreste genannt wie Methyl, Ethyl, n-Propyl, iso-Propyl, n-Butyl, iso-Butyl, tert.-Butyl und sek. Butyl.

Der niedere Acylrest leitet sich bevorzugt von aliphatischen Carbonsäuren mit bis zu 4 Kohlenstoffatome ab

wie beispielsweise Essigsäure, Propionsäure, Buttersäure u.a..

Die Salze der erfindungsgemäßen Verbindungen der Formel I sind Säureadditionssalze und leiten sich von üblicherweise verwendeten Säuren ab. Solche Säuren sind zum Beispiel anorganische Säuren,wie beispielsweise Chlorwasserstoffsäure, Salpetersäure, Phosphorsäure, Schwefelsäure, Bromwasserstoffsäure, Jodwasserstoffsäure, salpetrige Säure oder phosphorige Säure oder organische Säuren, wie beispielsweise aliphatische Mono- oder Dicarbonsäuren, phenylsubstituierte Alkancarbonsäuren, Hydroxyalkancarbonsäuren oder Alkendicarbonsäuren, aromatische Säuren oder aliphatische oder aromatische Sulfonsäuren. Physiologisch unbedenkliche Salze dieser Säuren sind daher z.B. das Sulfat, Pyrosulfat, Bisulfat, Sulfit, Bisulfit, Nitrat, Phosphat, Monohydrogenphosphat, Dihydrogenphosphat, Metaphosphat, Pyrophosphat, Chlorid, Bromid, Jodid, Fluorid, Acetat, Propionat, Decanoat, Caprylat, Acrylat, Formiat, Isobutyrat, Caproat, Heptanoat, Propiolat, Malonat, Succinat, Suberat, Sebacat, Fumarat, Maleat, Mandelat, Butin-1,4-dioat, Hexin-1,6-dioat, Benzoat, Chlorbenzoat, Methylbenzoat, Dinitrobenzoat, Hydroxybenzoat, Methoxybenzoat, Phthalat, Terephthalat, Benzolsulfonat, Toluolsulfonat, Chlorbenzolsulfonat, Xylolsulfonat, Phenylacetat, Phenylpropionat, Phenylbutyrat, Citrat, Lactat, 8-Hydroxybutyrat, Glycollat, Malat, Tartrat, Methansulfonat, Propansulfonat, Naphtalin-1-sulfonat oder Naphtalin-2-sulfonat.

Die Herstellung der erfindungsgemäßen Verbindungen erfolgt nach an sich bekannten Methoden.

Beispielsweise erfolgt die Herstellung der Verbindungen der allgemeinen Formel I dadurch, daß man eine Verbindung der allgemeinen Verbindung II

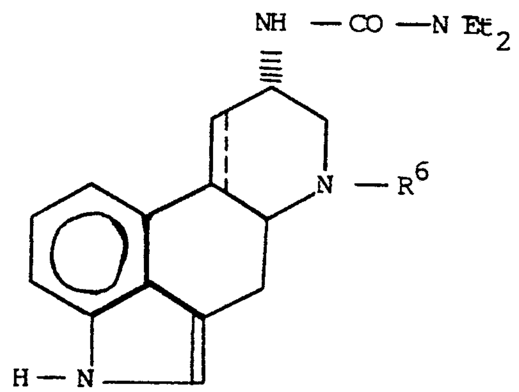

II

worin

$R^6$ niederes Alkyl ist, mit einer Verbindung der allgemeinen Formel III

Ar - X III

worin

Ar die oben genannte Bedeutung hat und X ein Halogen ist, aryliert und gegebenenfalls das Säureadditionssalz bildet.

Bei Einführung eines substituierten Ar-Restes können gewünschtenfalls die nachfolgend beschriebenen Folgereaktionen nach bekannten Methoden durchgeführt werden.

Beispielsweise gelangt man zu den Verbindungen der allgemeinen Formel I dadurch, daß man mit Verbindungen der allgemeinen Formel IIIa

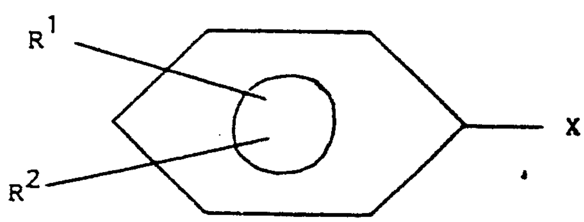

IIIa

worin

X Halogen bedeutet und

$R^1$ und

$R^2$ die oben genannte Bedeutung hat, aryliert und gewünschtenfalls anschließend

a) eine $NO_2$ -Gruppe reduziert und die so erhaltene $NH_2$ -Gruppe gegebenenfalls alkyliert oder acyliert oder diazotiert und gegen Wasserstoff, Hydroxy oder Halogen austauscht oder

b) eine Cyanogruppe verseift zum Carbonsäureamid oder $COOR^7$ , wobei $R^7$ die oben genannte Bedeutung hat, oder

c) eine Cyanogruppe reduziert zum Formylrest und gegebenenfalls anschließend in die $CH_2\ OR^8$ -Gruppe überführt oder zum-$CH_2\ NR^9\ R^{10}$ -Rest mit $R^8$ , $R^9$ und $R^{10}$ in der oben genannten Bedeutung reduziert und

anschließend die so erhaltenen Verbindungen der allgemeinen Formel I gewünschtenfalls in die physiologisch verträglichen Säureadditionssalze überführt.

In gleicher Weise kann zur Herstellung der I-Hetarylderivate verfahren werden.

Der Substituent X bedeutet insbesondere Fluor, Chlor und Brom.

Die Arylierung kann beispielsweise im basischen Milieu in inerten Lösungsmitteln bei Temperaturen von 0° C bis zum Siedepunkt des Reaktionsgemisches, bevorzugt bei Raumtemperatur, in 1 bis 20 Stunden vorgenommen werden.

Als Basen können Alkaliverbindungen wie beispielsweise Natrium-oder Kaliumhydroxid, Natriumhydrid, Natrium- oder Kaliumcarbonat zugesetzt werden; gegebenenfalls wird in Gegenwart von Phasentransferkatalysatoren wie beispielsweise Kronenäther wie 18-Krone-6, Dicyclohexyl-18-Krone-6, Dibenzo-18-Krone-6, Aliquat 336 oder Tetraalkylammoniumsalzen aryliert.

Die Reduktion der Nitrogruppe zur Aminogruppe erfolgt beispielsweise katalytisch in inerten Lösungsmitteln bei Raumtemperatur.

Vorzugsweise wird als Katalysator Palladium auf einem Träger wie Kohle oder Platin in fein verteilter Form verwendet; bei Verbindungen mit Halogen verwendet man als Katalysator vorzugsweise Raney-Nickel.

Für die Reduktion geeignete Lösungsmittel sind beispielsweise Alkohole wie Methanol, Ethanol oder Ether wie Diethylether, Tetrahydrofuran oder deren Mischungen.

Die Hydrierung kann unter Normaldruck oder $H_2$ -Druck vorgenommen werden.

Die Alkylierung der Aminogruppe kann durch Umsetzung mit Aldehyden in Gegenwart von reduzierenden Mitteln wie beispielsweise Hydrierung in Gegenwart von Palladium auf Kohle oder mit Natriumboranat in protischen Lösungsmitteln wie z.B. Alkoholen bei Raumtemperatur bis zum Siedepunkt des Reaktionsgemisches oder durch Umsetzung mit Alkylhalogeniden, z.B. Methyliodid, Ethyliodid u.a., erfolgen.

Die Acylierung der Amine kann durch Umsetzung mit Säurechloriden oder Säureanhydriden in Gegenwart von Basen wie Pyridin, Dimethylaminopyridin, Natrium- oder Kaliumhydroxiden, Natrium- oder Kaliumcarbonaten, gegebenenfalls unter Zusatz eines inerten Lösungsmittels vorgenommen werden. Die Diazotierung wie auch der Austausch der Aminogruppe gegen Wasserstoff und Hydroxy können nach dem literaturbekannten Sandmeyer-Verfahren erfolgen.

Zur Desaminierung wird beispielsweise die mit einem Nitrit intermediär erzeugt Diazoniumverbindung in Gegenwart von Cu-I-oxid und hypophosphoriger Säure reduktiv bei erhöhter Temperatur verkocht.

Durch Verkochung der Diazoniumsalze in wässriger Lösung können die entsprechenden Phenole hergestellt werden.

Zur Einführung der Halogene können die intermediär gebildeten Diazoniumsalze mit Cu-I-chlorid oder Cu-I-bromid in Gegenwart der entsprechenden Säure (Salzsäure oder Bromwasserstoffsäure) oder mit Kaliumiodid umgesetzt werden.

Die Einführung von Fluor gelingt beispielsweise durch die Balz-Schiemann-Reaktion des Diazoniumtetrafluorborates.

Die Verseifung der Cyanogruppe zum Carbonsäureamid kann durch Umsetzung mit 96 % $H_2SO_4$ bei Raumtemperatur erfolgen.

Durch Umsetzung der Cyanogruppe mit starken Säuren wie Schwefelsäure, Salzsäure oder mit starken Basen wie Natrium-oder Kaliumhydroxid bei Siedetemperatur des Reaktionsgemisches in inerten Lösungsmitteln können die entsprechenden Carbonsäuren erhalten werden.

Die Darstellung der Ester aus Nitrilen kann durch Addition von Alkoholen in Gegenwart von wasserfreier Salzsäure und anschließender Verseifung mit Wasser erfolgen. Im allgemeinen arbeitet man unter Eiskühlung.

Die Reduktion der Cyanogruppe zur Formylgruppe kann mit DIBAH in aprotischen Lösungsmitteln wie z.B. Kohlenwasserstoffen wie Toluol oder Ether, wie THF bei Temperaturen von - 70 °C bis 20 °C erfolgen.

Durch Weiterreduktion der Formylgruppe beispielsweise mit DIBAH, Natriumborhydrid oder Lithiumaluminiumhydrid in inerten Lösungsmitteln wie z.B. Kohlenwasserstoffen, Alkoholen, Ethern kann man die entsprechenden Alkohole erhalten, die alkyliert oder acyliert werden können.

Die Alkylierung kann in üblicher Weise vorgenommen werden; vorzugsweise wird mit Alkyliodiden in Gegenwart starker Basen wie beispielsweise Natriumhydrid umgesetzt.

Die Acylierung kann üblicherweise mit Säurechloriden und Säureanhydriden, wie oben beschrieben, erfolgen.

Durch Reduktion der Cyanogruppe mit Wasserstoff wie z.B. mit $LiAlH_4$ oder in Gegenwart von Palladium gegebenenfalls auf Trägern kann man Aminomethylverbindungen erhalten, die anschließend wie bei den Aminoverbindungen beschrieben, alkyliert oder acyliert werden können.

Zweckmäßigerweise werden die oben beschriebenen Reaktionen unter Inertgasatmosphäre wie beispielsweise unter Stickstoff oder Argon vorgenommen.

Bei Herstellung der Salze wird die erhaltene Verbindung in wenig Methanol gelöst und mit einer konzentrierten Lösung der gewünschten Säure in Methanol bei Raumtemperatur versetzt.

Im Vergleich zu bekannten, in 1-Stellung nicht substituierten Ergolinen, wie zum Beispiel dem Lisurid oder dem Tergurid, zeichnen sich die erfindungsgemäßen Verbindungen der allgemeinen Formel I durch eine zentral antidopaminerge Wirksamkeit und/oder $\alpha_2$ -rezeptorblockierende Wirkung aus.

Die zentrale Dopaminrezeptorblockade wurde in einem Interaktionstest mit dem Dopaminrezeptoragonisten Apomorphin an Mäusen nach einmaliger i.p.-Vorbehandlung dargestellt (Parameter: Aufhebung der durch Apomorphin 5 mg/kg i.p. verursachten Hypothermie). Männliche NMRI-Mäuse wurden mit

verschiedenen Dosen der Substanz, die selbst nicht die Thermoregulation der Versuchstiere beeinflußen bzw. mit Trägermedium vorbehandelt. 30 Minuten später erhielten alle Tiere Apomorphin 5 mg/kg i.p. 60 Minuten nach Zugabe von Substanz bzw. Trägermedium (= 30 Min nach Apomorphin) wurde die Rektaltemperatur mit Hilfe einer Thermosonde gemessen. Während die mit Trägermedium vorbehandelten Mäuse eine Hypothermie aufwiesen, war an mit Substanz vorbehandelten Tieren der körpertemperatursenkende Effekt des Apomorphin dosisabhängig aufgehoben.

Die zentrale $\alpha_2$ -Repzeptorblockade wurde in einem Interaktionstest mit dem $\alpha_2$ -Rezeptoragonisten Clonidin an Mäusen nach einmaliger i.p.-Vorbehandlung dargestellt (Parameter: Aufhebung der durch Clonidin 0,1 mg/kg i.p. verursachten Hypothermie). Männliche NMRI-Mäuse wurden mit verschiedenen Dosen der Substanz, die selbst nicht die Thermoregulation der Versuchstiere beeinflussen, bzw. mit Trägermedium vorbehandelt. 30 Minuten später erhielten alle Tiere Clonidin 0,1 mg/kg i.p., 60 Minuten nach Zugabe von Substanz bzw. Trägermedium ( = 30 Minuten nach Clonidin) wurde die Rektaltemperatur mit Hilfe einer Thermosonde gemessen. Während die mit Trägermedium vorbehandelten Mäuse eine Hypothermie aufwiesen, war an mit Substanz vorbehandelten Tieren der körpertemperatursenkende Effekt des Clonidin dosisabhängig aufgehoben.

Aufgrund dieser Befunde können die erfindungsgemäßen Verbindungen daher als Neuroleptika zur Behandlung von Psychosen des schizophrenen Formenkreises oder als Antidepressiva verwendet werden.

Aufgrund dieser Befunde können die erfindungsgemäßen Verbindungen daher beispielsweise als Antidepressiva verwendet werden.

Zur Verwendung der erfindungsgemäßen Verbindungen als Arzneimittel werden diese in die Form eines pharmazeutischen Präparats gebracht, das neben dem Wirkstoff ein für die enterale oder parentale Applikation geeignete pharmazeutische, organische oder anorganische inerte Trägermaterialien, wie zum Beispiel Wasser, Gelatine, Gummi arabicum, Milchzucker, Stärke, Magnesiumstearat, Talk, pflanzliche Öle, Polyalkylenglykole usw. enthält. Die pharmazeutischen Präparate können in fester Form, zum Beispiel als Tabletten, Dragees, Suppositorien, Kapseln oder in flüssiger Form, zum Beispiel als Lösungen, Suspensionen oder Emulsionen vorliegen. Gegebenenfalls enthalten sie darüberhinaus Hilfsstoffe wie Konservierungs, Stabilisierungs-, Netzmittel oder Emulgatoren, Salze zur Veränderung des osmotischen Drucks oder Puffer.

Die Herstellung der Ausgangsverbindungen ist bekannt oder erfolgt nach bekannten Methoden.

Die nachfolgenden Beispiele sollen das erfindungsgemäße Verfahren erläutern.

<u>Beispiel 1</u>

<u>1,1-Diethyl-3-(6-methyl-1-(4-nitrophenyl)-8 $\alpha$-ergolinyl)-harnstoff</u>

In 200 ml Tetrahydrofuran löst man 3,4 g Tergurid (10 mMol), gibt 0,4 g Tetrabutylammoniumhydrogensulfat, 5,0 g festes, pulverisiertes Kaliumhydroxid und 5,3 ml 4-Fluornitrobenzol (50 mMol) zu. Man rührt 2 Stunden bei Raumtemperatur kräftig unter einer Argonatmosphäre und verteilt dann, gegebenenfalls nach Abziehen eines Teils des Lösungsmittels, zwischen Essigester und gesättigter Kochsalzlösung. Die organische Phase wird mit Natriumsulfat getrocknet und so weit eingedampft, bis sich Kristalle bilden. Nach Stehen im Eisbad trennt man die Kristalle ab.

Ausbeute 4,4 g (95 %), $[\alpha]_D$ = - 37 ° (0,5 % in Chloroform)

<u>Beispiel 2</u>

<u>3-(1-(4-Aminophenyl)-6-methyl-8 $\alpha$-ergolinyl)-1,1-diethyl-harnstoff</u>

Man löst 2,3 g der nach Beispiel 1 hergestellten Verbindung (5 mMol) in 35 ml Methanol und 35 ml THF, fügt 1,1 g Palladium auf Kohle (10 %ig) zu und hydriert bei Raumtemperatur und Normaldruck. Nach 30 Minuten wird vom Katalysator abfiltriert, das Filtrat eingedampft und aus Essigester und Diisopropylether kristallisiert.

Ausbeute 1,9 g (88 % d. Th.), $[\alpha]_D$ = - 16 ° (0,5 % in Chloroform).

<u>Beispiel 3</u>

<u>1,1-Diethyl-3-(6-methyl-1-phenyl-8 $\alpha$ -ergolinyl)-harnstoff</u>

432 mg der nach Beispiel 2 hergestellten Verbindung (1 mMol) werden in 8 ml Wasser suspendiert, mit 3,8 ml konz. Salzsäure und 8 ml Wasser versetzt. Unter Kühlung im Eisbad wird 10 Minuten gerührt, dann 74 mg Natriumnitrit (1,06 mMol) in 1 ml Wasser zugegeben und 30 Minuten gerührt. Danach werden 5 ml 60 %ige Hypophoshorige Säure und eine Spatelspitze Kupfer-I-oxid zugegeben und eine Stunde bei Raumtemperatur gerührt. Die Mischung wird mit 5 ml konz. Ammoniaklösung alkalisch gemacht und mit Methylenchlorid ausgeschüttelt. Die organische Phase wird getrocknet, eingedampft und der Rückstand an Kieselgel mit Methylenchlorid und Methanol chromatographiert. Man isoliert 365 mg, die aus Essigester und Diisopropylether kristallisiert werden.

Ausbeute 223 mg (36 % d. Th.), $[\alpha]_D$ = - 13 ° (0,5 % in Chloroform).

Analog Beispiel 1 werden dargestellt:

Beispiel 4

1,1-Diethyl-3-(6-methyl-1-pyrimidin-2-yl-8 α-ergolinyl)-harnstoff
aus Tergurid und 2-Chlorpyridin.

Beispiel 5

1,1-Diethyl-3-(6-methyl-1-pyrazinyl-8 α-ergolinyl)-harnstoff
aus Tergurid und 2-Chlorpyrazin.

Beispiel 6

1,1-Diethyl-3-(6-methyl-1-thiazol-2-yl-8 α-ergolinyl)-harnstoff
aus Tergurid und 2-Brom-thiazol.

**Patentansprüche**

1. Verbindungen der allgemeinen Formel I

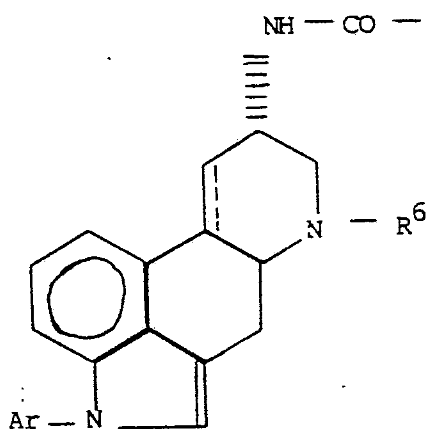

I .

worin
Ar für einen gegebenenfalls substituierten Aryl- oder Hetarylrest steht und
$C_9$ ===== $C_{10}$ eine Einfach- oder Doppelbindung sein kann sowie deren Säureadditionssalze.

2. Verbindungen gemäß Anspruch 1, worin Ar für den Phenylrest

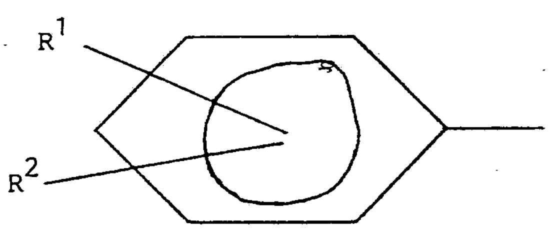

steht,
worin
$R^1$ und $R^2$ jeweils Wasserstoff, Trifluormethyl, Nitro, Cyano, Halogen, Formyl, Aminocarbonyl, niederes Alkyl, $NR^3R^4$ , wobei $R^3$ and $R^4$ jeweils Wasserstoff, niederes Alkyl oder niederes Acyl darstellen,
$OR^5$ , wobei $R^5$ Wasserstoff, niederes Alkyl oder niederes Acyl bedeutet,
$COOR^7$ , wobei $R^7$ Wasserstoff oder niederes Alkyl ist,
$CH_2$ $OR^8$ , wobei $R^8$ Wasserstoff, niederes Alkyl oder niederes Acyl ist
oder
$CH_2$ $NR^9$ $R^{10}$ , wobei $R^9$ und $R^{10}$ jeweils Wasserstoff, niederes Alkyl oder niederes Acyl darstellt,
bedeuten.

3. 1,1-Diethyl-3-(6-methyl-1-(4-nitrophenyl)-8 α-ergolinyl)-harnstoff
3-(1-(4-Aminophenyl)-6-methyl-8 α-ergolinyl)-1,1-diethyl-harnstoff
1,1-Diethyl-3-(6-methyl-1-phenyl-8 α-ergolinyl)-harnstoff

1,1-Diethyl-3-(6-methyl-1-pyrimidin-2-yl-8 α-ergolinyl-harnstoff
1,1-Diethyl-3-(6-methyl-1-pyrazinyl-8 α-ergolinyl)-harnstoff
1,1-Diethyl-3-(6-methyl-1-thiazol-2-yl-8 -ergolinyl)-harnstoff

4. Verfahren zur Herstellung der Verbindungen der allgemeinen Formel I dadurch, daß man eine Verbindung der allgemeinen Formel II

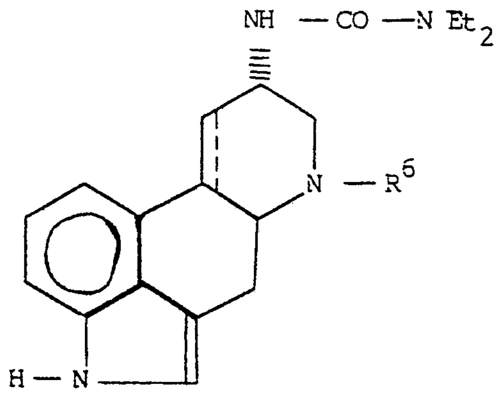

II

worin
$R^6$ niederes Alkyl ist, mit einer Verbindung der allgemeinen Formel III
Ar - X III
worin
Ar die oben genannte Bedeutung hat und X ein Halogen ist, aryliert und gegebenenfalls das Säureadditionssalz bildet.

5. Verfahren gemäß Anspruch 4, dadurch, daß man Verbindungen der allgemeinen Formel IIIa

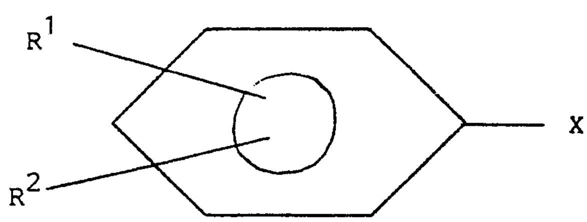

IIIa

worin
X Halogen bedeutet und
$R^1$ Trifluormethyl, Nitro, Cyano, Halogen oder $COOR^7$ mit $R^7$ in der Bedeutung von Wasserstoff oder nieder Alkyl oder Halogen ist und
$R^2$ die oben genannte Bedeutung hat, aryliert und gewünschtenfalls anschließend
a) eine $NO_2$ -Gruppe reduziert und die so erhaltene $NH_2$ -Gruppe gegebenenfalls alkyliert oder acyliert oder diazotiert und gegen Wasserstoff, Hydroxy oder Halogen austauscht oder
b) eine Cyanogruppe verseift zum Carbonsäureamid oder $COOR^7$ wobei $R^7$ die oben genannte Bedeutung hat, oder
c) eine Cyanogruppe reduziert zum Formylrest und gegebenenfalls anschließen in die $CH_2$ $OR^8$ -Gruppe überführt oder zum$CH_2$ $NR^9$ $R^{10}$ -Rest mit $R^8$ , $R^9$ und $R^{10}$ in der oben genannten Bedeutung reduziert und
anschließend die so erhaltenen Verbindungen der allgemeinen Formel I gewünschtenfalls in die physiologisch verträglichen Säureadditionssalze überführt.

6. Arzneimittel, bestehend aus einer oder mehreren Verbindungen gemäß Anspruch 1 und den üblichen Hilfs- und Trägerstoffen.

Europäisches Patentamt

**EUROPÄISCHER RECHERCHENBERICHT**

Nummer der Anmeldung

EP 87 73 0073

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | GB-A-2 116 548 (SPOFA SPOJENE PODNIKY PRO ZDRAVOTNICKOU VYROBU) * Ansrpüche 1, 6; Zusammenfassung * --- | 1,4,6 | C 07 D 457/12<br>A 61 K 31/48 |
| A | EP-A-0 118 848 (SCHERING AG) * Ansprüche 2, 7 * ----- | 1,6 | |
| | | | **RECHERCHIERTE SACHGEBIETE (Int. Cl.4)** |
| | | | C 07 D 457/00 |

Der vorliegende Recherchenbericht wurde für alle Patentansprüche erstellt.

| Recherchenort | Abschlußdatum der Recherche | Prüfer |
|---|---|---|
| BERLIN | 06-10-1987 | HASS C V F |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82